# EUROPÄISCHE PATENTSCHRIFT

(11) **EP 3 225 371 B1**
(45) Veröffentlichungstag und Bekanntmachung des Hinweises auf die Patenterteilung: **12.09.2018**
(21) Anmeldenummer: 16163609.7
(22) Anmeldetag: 01.04.2016
(51) Int. Cl.: B28B 11/04, B28B 17/00, G01N 15/08, G01N 33/38

(54) **VERFAHREN UND VORRICHTUNG ZUM VERSEHEN EINES BETONWARENPRODUKTES MIT EINEM BESCHICHTUNGSMATERIAL**
METHOD AND DEVICE FOR PROVIDING A CONCRETE PRODUCT WITH A COATING MATERIAL
PROCEDE ET DISPOSITIF DESTINES A DOTER UN PRODUIT EN BETON D'UN MATERIAU DE REVETEMENT

(43) Veröffentlichungstag der Anmeldung: 04.10.2017
(73) Patentinhaber: Omnicon GmbH, 24976 Flensburg-Handewitt (DE)
(72) Erfinder: Biallas, Thorsten, 24943 Flensburg (DE)
(74) Vertreter: Uexküll & Stolberg

(56) Entgegenhaltungen:
- EP-A1- 1 800 817
- WO-A1-98/29731
- WO-A2-2009/034085
- DE-A1- 3 628 955
- DE-A1-102012 001 805

## Beschreibung

Die vorliegende Erfindung betrifft ein Verfahren und eine Vorrichtung zum Versehen eines Betonwarenproduktes mit einem Beschichtungsmaterial wie insbesondere einem Oberflächenschutzprodukt.

Betonwarenprodukte werden als Fertigprodukte zu ihrem Einsatzort, wie etwa zu einer Baustelle, geliefert. Ein Beispiel für Betonwarenprodukte sind Pflastersteine. Zur Herstellung von Betonwarenprodukten wird zunächst Beton nach einer vorgegebenen Rezeptur gemischt. Anschließend wird der Beton in eine Maschine eingebracht, in der er in die Form der gewünschten Betonwarenprodukte gepresst und dabei verdichtet wird. Im Falle von beispielsweise Pflastersteinen wird eine sogenannte Steinformmaschine verwendet, um die Pflastersteine zu pressen. Die durch den Verdichtungsprozess entstehenden Rohlinge sind noch weich und verarbeitbar. Nach dem Verdichtungsprozess werden die Rohlinge für ungefähr ein bis zwei Tage in einer Trockenkammer angeordnet und anschließend für einen weiteren Zeitraum gelagert. In der Trockenkammer und während der anschließenden Lagerung erhärtet der Beton, und die Rohlinge werden auf diese Weise zu den fertigen Betonwarenprodukten.

Der Herstellungsprozess der Betonwarenprodukte kann grob in zwei Phasen eingeteilt werden. Die erste Phase bis zum Erhärten des Betons, d.h. die Phase, in der die Rohlinge noch weich und verarbeitbar sind, wird im Allgemeinen als Frischbetonphase bezeichnet. Entsprechend werden die noch weichen und verarbeitbaren Rohlinge als Frischbetonprodukte bezeichnet. Die Frischbetonprodukte sind demnach die fertig gepressten und verdichteten Vorformen der fertigen Betonwarenprodukte. An die Frischbetonphase schließt sich die Festbetonphase an, in der der Beton erhärtet ist. Entsprechend werden die fertigen Betonwarenprodukte auch als Festbetonprodukte bezeichnet. Demnach wird zur Herstellung eines bestimmten Betonwarenproduktes zunächst aus dem in geeigneter Weise gemischten Beton ein entsprechendes Frischbetonprodukt hergestellt, das durch Aushärten zu einem Festbetonprodukt wird, das das gewünschte Betonwarenprodukt darstellt.

Betonwarenprodukte kommen regelmäßig in Bereichen zum Einsatz, in denen sie nicht nur starken mechanischen Belastungen, sondern auch weiteren äußeren Einflüssen, wie Wasser, Schnee, Licht, Hitze oder Frost, ausgesetzt sind. Beispiele hierfür sind Dachsteine oder auch Pflastersteine, Bordsteine oder sonstige Betonwarenprodukte, die im Garten- und/oder Wegebau eingesetzt werden. Als Baumaterialien müssen sie dennoch eine lange Lebensdauer aufweisen und ferner bestimmten Qualitätsnormen genügen. Daher ist es von großer Bedeutung, zuverlässig die Qualität der auf die obige Weise hergestellten Betonwarenprodukte bestimmen zu können. So wird häufig ein Festigkeitswert, die Rohdichte, die Frostbeständigkeit, die Porosität oder ein anderes Qualitätsmaß der fertigen Betonwarenprodukte gemessen, um die Qualität des Herstellungsprozesses und der durch diesen hergestellten Betonwarenprodukte zu bewerten und auf Basis dieser Bewertung gegebenenfalls Korrekturen an den Prozessparametern vorzunehmen.

Das Erhärten des Frischbetonproduktes zu dem Festbetonprodukt erfolgt dabei über einen relativ langen Zeitraum. So erhält der Beton seine Festigkeit dadurch, dass die Klinkerbestandteile des einen Teil der Betonmischung bildenden Zements auskristallisieren. Durch das Auskristallisieren entstehen kleine Kristallnadeln, die sich fest ineinander verzahnen. Da sich das Kristallwachstum jedoch über einen Zeitraum von einigen Monaten fortsetzt, wird die endgültige Festigkeit erst lange nach dem Formen und Verdichten bzw. der Herstellung des Frischbetonproduktes erreicht. In der DIN 1164/EN 1338 ist die Normfestigkeit als genormtes Qualitätsmaß für die Festbetonprodukte als die Festigkeit definiert, die unter normalen Temperatur- und Feuchtigkeitsbedingungen nach 28 Tagen erreicht wird. In ähnlicher Weise werden auch aussagekräftige Werte weiterer Qualitätsmaße, wie zum Beispiel die Rohdichte, die Frostbeständigkeit, oder die abschließende Porosität erst nach diesem Zeitraum erreicht. Daher werden zur Qualitätskontrolle einzelne fertige Betonwarenprodukte gewöhnlich erst nach 28 Tagen auf ihre Qualität überprüft. Erst dann wird festgestellt, ob die Betonwarenprodukte den gewünschten Anforderungen entsprechen oder nicht.

Die charakteristischen Eigenschaften der fertigen Betonwarenprodukte, wie zum Beispiel ihre Festigkeit oder die Porosität, werden maßgeblich durch die Einsatzstoffe und ihre relativen Mengen in der anfänglichen Betonmischung sowie durch die Verdichtung bei der Herstellung des Rohlings bzw. Frischbetonproduktes bestimmt. So können bei der Verdichtung Verdichtungsunterschiede auftreten, die zu unterschiedlichen Güten führen. Dasselbe gilt für Variationen in den durch die gewählte Rezeptur vorgegebenen Rohstoffen. So kann insbesondere das auch als W/Z-Wert bezeichnete Wasser/Zement-Verhältnis, das durch direkt zugegebenes Wasser und in den übrigen Rohstoffen enthaltenes Wasser bestimmt wird, im Rahmen einer gegebenen Rezeptur schwanken. Daher ist die Frischbetonkonsistenz vor der Herstellung festzulegen und möglichst genau einzuhalten. Im Fall von Wasser werden regelmäßig Wasserdosiergeräte in den Mischern eingesetzt, um die Variation des Wassergehalts zu begrenzen.

Die Porosität des Frischbetonproduktes bzw. des späteren Festbetonprodukts ist eine wichtige Eigenschaft des Betons. Die im Beton vorhandenen Poren können sich zu Kapillarkanälen vernetzen, die bis an die Außenfläche des Betonprodukts reichen. Durch diese Kapillare ist ein Transport von Feuchtigkeit und mit der Feuchtigkeit gegebenenfalls der Transport von Schadstoffen in das Betonprodukt möglich. Durch einen Wechsel von Feucht- und Trockenzyklen, wie er beispielsweise bei im Außenbereich eingesetzten Pflastersteinen vorkommt, findet dann eine Aufkonzentration von Schadstoffen im Betonprodukt statt, welches die Lebensdauer stark verringern kann. Desweiteren kann z.B. Regenwasser, das über die Poren in einen Pflasterstein eingedrungen ist, bei Gefrieren den Stein durch die Volumenvergrößerung des Wassers zerstören. Dieser Schädigungseffekt von Wasser wird noch einmal in der Kombination von Tausalzen, wie sie auf Straßen und Wegen eingesetzt werden, verstärkt. Zur Vermeidung der genannten Schädigungseffekte des Betonprodukts werden Oberflächenschutzprodukte bereits auf die Außenflächen von Frischbetonprodukten in einem Zwischenschritt nach dem Formen und Verdichten aufgebracht. Die Oberflächenschutzprodukte schließen dabei die Poren und versiegeln die Oberfläche. Dadurch wird ein Eindringen von Schadstoffen, insbesondere von Feuchtigkeit, in das Betonprodukt verhindert. Die Druckschrift WO2009/034085 A2 offenbart ein Verfahren und eine Vorrichtung zum Versehen einer Anzahl von Betonwarenprodukten während der Frischbetonphase mit einem Beschichtungsmaterial.

Die Druckschrift EP1800817 A1 offenbart, dass an einem Test-Frischbetonprodukt aus einem Zwischenschritt des Herstellungsprozesses nach dem Formen und Verdichten Messungen vorgenommen werden, um Messwerte zu ermitteln, wobei die Messwerte ein Maß für die Rohdichte und die Gaspermeabilität des Test-Frischbetonproduktes umfassen.

Im Stand der Technik wird die auf ein Frischbetonprodukt aufzubringende Menge eines Oberflächenschutzproduktes von dem Maschinenführer entschieden.

Dasselbe gilt auch für andere Materialien bzw. Beschichtungsmaterialien, die ggf. auf die Außenflächen von Frischbetonprodukten aufgebracht werden.

Um einen optimalen und auch wirtschaftlich ertragreichen Herstellungsprozess zu erreichen ist es wichtig, dass die eingesetzte Menge des Oberflächenschutzproduktes bzw. Beschichtungsmaterials pro Frischbetonprodukt so groß wie nötig, um die gewünschte Qualität zu erreichen, aber auch so klein wie möglich ist, um Kosten zu sparen. Eine Bestimmung genau dieser Menge durch den Maschinenführer setzt jedoch viel Erfahrung voraus und ist dennoch kaum zuverlässig möglich. Um dieses Problem zu umgehen wird daher tendenziell eine übermäßige Menge des Oberflächenschutzproduktes bzw. Beschichtungsmaterials pro Frischbetonprodukt verwendet, um so sicherzugehen, dass auch bei individuellen Variationen der Porosität der Oberfläche der gewünschte Oberflächenschutz bzw. Zweck des Beschichtungsmaterials durch die Beschichtung erreicht wird. Ein derartiger übermäßiger Einsatz des Oberflächenschutzproduktes bzw. Beschichtungsmaterials ist aber mit erhöhten Kosten verbunden.

Es ist daher Aufgabe der Erfindung, ein Verfahren und eine Vorrichtung zum Versehen eines Betonwarenproduktes mit einem Beschichtungsmaterial, wie insbesondere einem Oberflächenschutzprodukt, bereitzustellen, mit denen zuverlässig, einfach, schnell, individuell und kosteneffizient die Beschichtung eines Betonwarenproduktes vorgenommen werden kann und mit denen die genannten Nachteile beseitigt werden. Ferner soll die Vorrichtung robust sein, um sie in einer rauen Produktionsumgebung betreiben zu können.

Zur Lösung dieser Aufgabe dienen die Merkmale der Patentansprüche 1 und 7. Vorteilhafte Ausführungsformen des Verfahrens und der Vorrichtung sind Gegenstand der jeweils zugehörigen Unteransprüche.

Nach der vorliegenden Erfindung ist ein Verfahren zum Versehen einer Anzahl von Betonwarenprodukten mit einem Material bzw. Beschichtungsmaterial vorgesehen, bei dem während eines Prozesses zur Herstellung von Betonwarenprodukten, der einen einer Frischbetonphase entsprechenden Zwischenschritt nach Formen und Verdichten aufweist, in dem die Betonwarenprodukte als Frischbetonprodukte vorliegen, in der Frischbetonphase durch einen Sprühvorgang mittels einer Sprühvorrichtung ein Materials bzw. Beschichtungsmaterial auf eine Oberfläche oder Oberflächen einer Anzahl der Frischbetonprodukte aufgebracht wird, die der Anzahl von Betonwarenprodukten entsprechen. Mit anderen Worten werden die Frischbetonprodukte, die die Anzahl von Frischbetonprodukten bilden, im weiteren Verlauf des Prozesses zu den Betonwarenprodukten, die die Anzahl von Betonwarenprodukten bilden. Die Anzahl von Frischbetonprodukten kann ein oder mehrere Frischbetonprodukte umfassen. Entsprechend kann die Anzahl von Betonwarenprodukten ein oder mehrere Betonwarenprodukte umfassen.

Das Verfahren weist außerdem den Schritt auf, dass ein Maß für die Porosität eines Frischbetonproduktes aus der Frischbetonphase über eine Messeinrichtung bestimmt wird. Dieses Frischbetonprodukt, das auch als Test-Frischbetonprodukt bezeichnet werden kann, kann dabei zu der Anzahl der Frischbetonprodukte gehören, muss es aber nicht. Das bedeutet, dass das Frischbetonprodukt, an dem die Messung vorgenommen wird, dem Prozess auch entnommen und ggf. entsorgt werden kann, so dass später kein Betonwarenprodukt aus ihm entsteht. Es ist außerdem darauf hinzuweisen, dass die Messung auch an mehr als einem Frischbetonprodukt vorgenommen werden kann. Dann kann das Maß für die Porosität ein Maß für die Porosität der Gesamtheit dieser Frischbetonprodukte sein, an denen die Messung vorgenommen worden ist, und dieses Maß kann im Folgenden verwendet werden.

Außerdem weist das Verfahren den Schritt auf, den Sprühvorgang des Materials bzw. Beschichtungsmaterials auf die Anzahl von Frischbetonprodukten in Abhängigkeit von dem bestimmten Maß für die Porosität durch Steuerung mindestens eines Verfahrensparameters zur Einstellung einer Auftragsmenge des Materials bzw. Beschichtungsmaterials durchzuführen. Da das Frischbetonprodukt, an dem die Messung vorgenommen worden ist, aus demselben Prozess und daher bevorzugt aus derselben Produktionsmenge oder Charge stammt wie die Anzahl von Frischbetonprodukten, ist seine Porosität für diejenige der Anzahl von Frischbetonprodukten charakteristisch.

Typische Verfahrensparameter können beispielsweise die Durchflussmenge oder Durchflussgeschwindigkeit des Materials bzw. Beschichtungsmaterials durch eine Sprühvorrichtung während des Sprühvorgangs, die Geschwindigkeit mit der die Sprühvorrichtung über die Anzahl von Frischbetonprodukten bzw. deren Oberflächen während des Sprühvorgangs bewegt wird oder eine Kombination aus beiden sein. Die auf das jeweilige Frischbetonprodukt aufgebrachte Auftragsmenge kann dabei insbesondere die Auftragsmenge pro Flächeninhalt sein.

Das Material bzw. Beschichtungsmaterial kann bevorzugt ein Oberflächenschutzprodukt sein. Ein solches Oberflächenschutzprodukt stellt für die den Frischbetonprodukten entsprechenden Betonwarenprodukte einen Schutz bereit. Es kann zum Beispiel eine Beschichtung, Imprägnierung und/oder Versiegelung bewirken. Das Material bzw. Beschichtungsmaterial kann aber auch ganz allgemein ein Material bzw. Beschichtungsmaterial sein, das einen oder mehrere funktionelle Inhaltsstoffe aufweist, die eine oder mehrere Funktionen erfüllen. Zum Beispiel kann das Material bzw. Beschichtungsmaterial auch eine Farbe sein.

In einer bevorzugten Ausgestaltung, in der das Frischbetonprodukt, an dem die Messung durchgeführt wird, auch zu einem Betonwarenprodukt wird, ist das Verfahren anders ausgedrückt ein Verfahren zum Versehen eines Betonwarenproduktes mit einem Beschichtungsmaterial, bei dem während eines Prozesses zur Herstellung von Betonwarenprodukten ein Beschichtungsmaterial auf eine Oberfläche eines Frischbetonproduktes durch einen Sprühvorgang mittels einer Sprühvorrichtung aufgebracht wird, wobei das Aufbringen in einem Zwischenschritt des Prozesses nach dem Formen und Verdichten in der Frischbetonphase erfolgt. Es umfasst dabei die folgenden Schritte:
- Bestimmen eines Maßes für die Porosität des Frischbetonproduktes über eine Messeinrichtung,
- Durchführen des Sprühvorgangs des Beschichtungsmaterials auf das Frischbetonprodukt in Abhängigkeit von dem bestimmten Maß für die Porosität durch Steuerung mindestens eines Verfahrensparameters zur Einstellung einer Auftragsmenge des Beschichtungsmaterials. Das Frischbetonprodukt kann dabei zu einer größeren Anzahl von Frischbetonprodukten gehören, die gleichzeitig verarbeitet werden und für die derselbe Sprühvorgang durchgeführt wird.

Mit anderen Worten wird in dieser Ausgestaltung in einem ersten Verfahrensschritt die Porosität eines Frischbetonproduktes in einem Zwischenschritt des Herstellungsprozesses nach dem Formen und Verdichten gemessen. In einem anschließenden Verfahrensschritt wird der Sprühvorgang des Beschichtungsmaterials auf das Frischbetonprodukt in Abhängigkeit von dem bestimmten Maß für die Porosität durch Steuerung mindestens eines Verfahrensparameters durchgeführt. Die Steuervorrichtung stellt dabei die optimale Auftragsmenge des Beschichtungsmaterial sein. Typische Verfahrensparameter können beispielsweise die Durchflussmenge oder Durchflussgeschwindigkeit des Beschichtungsmaterials durch eine Sprühvorrichtung während des Sprühvorgangs, die Geschwindigkeit mit der die Sprühvorrichtung über ein Frischbetonprodukt während des Sprühvorgangs bewegt wird oder eine Kombination aus beiden sein. Die auf das Frischbetonprodukt aufgebrachte Auftragsmenge kann dabei insbesondere die Auftragsmenge pro Flächeninhalt sein.

In jedem Fall hat das erfindungsgemäße Verfahren den Vorteil, dass durch eine individuelle Messung der Porosität z.B. eines einzelnen Frischbetonproduktes oder kleinerer Produktionschargen die Variationen der Porosität bei der Bestimmung der eingesetzten Menge des Beschichtungsmaterials berücksichtigt werden kann. Diese Berücksichtigung der individuellen Porosität erlaubt eine kosteneffiziente Anpassung der eingesetzten Menge des Beschichtungsmaterials. Zudem ist der hohe Grad der Automation weit weniger arbeitsintensiv als eine manuelle Bestimmung der Auftragsmenge, was ebenfalls Personalkosten senken kann. Desweiteren ist die automatisierte, objektive Messung und Bestimmung der Auftragsmenge einfacher, schneller und zuverlässiger als eine manuelle, von den Fähigkeiten und Erfahrungen des jeweiligen Maschinenführers abhängige Bestimmung der Auftragsmenge.

In einer Ausführungsform des Verfahrens ist das Maß für die Porosität eine Gaspermeabilität und bevorzugt die Permeabilität gegenüber Luft. Die Gaspermeabilität ist ein Maß für die Gefügewerte oder auch die Nutzporosität des Frischbetonproduktes. Die Gaspermeabilität ist als Permeabilität für ein bestimmtes Gas oder Gasgemisch zu verstehen, da sich die Permeabilität im Allgemeinen für verschiedene Gase bzw. Gasgemische unterscheidet. Für die späteren Messungen muss daher im Allgemeinen stets dasselbe Gas bzw. Gasgemisch verwendet werden oder eine an die unterschiedlichen verwendeten Gase angepasste Zuordnungstabelle oder Zuordnungsfunktion zwischen der gemessenen Gaspermeabilität und der Porosität des Frischbetonprodukts verwendet werden. Das genaue Messverfahren ist dabei nicht von Bedeutung. Es kommt lediglich darauf an, dass stets dasselbe Messverfahren in demselben Verfahrensschritt, d.h. an vergleichbaren Frischbetonprodukten und Positionen auf den Frischbetonprodukten, durchgeführt wird.

Dabei ist es bevorzugt, wenn die Gaspermeabilität die Permeabilität gegenüber Luft ist. Diese Messung lässt sich auf besonders einfache, schnelle und kostengünstige Weise realisieren, da mit der normalen Atmosphäre gearbeitet werden kann und der Einsatz besonderer Gase nicht erforderlich ist. Es ist aber zu bedenken, dass in diesem Fall für die Genauigkeit der Messung eine obere Grenze existiert, da Beton gegenüber dem in der Luft enthaltenen Kohlendioxid nicht inert ist. Reicht die Genauigkeit der Messung mit Luft nicht aus, so können Gase wie Sauerstoff oder Stickstoff verwendet werden.

In einer bevorzugten Ausführungsform des Verfahrens wird die Gaspermeabilität gemessen, indem entlang einer Außenfläche des Frischbetonproduktes, für das das Maß für die Porosität bestimmt wird, eine Druckdifferenz hergestellt und ein Maß für den durch die Druckdifferenz bewirkten Gasdurchfluss durch das Frischbetonprodukt bestimmt wird. Mit anderen Worten wird ein Bereich der Oberfläche des Frischbetonproduktes, für das das Maß für die Porosität bestimmt wird, mit einem Überdruck oder einem Unterdruck beaufschlagt und eine Größe gemessen, die für das durch das Frischbetonprodukt gepresste oder gesogene Gas charakteristisch ist. Die Gaspermeabilität ist nur von dem verwendeten Gas und den Eigenschaften des Frischbetonproduktes abhängig, wenn bei der Messung laminare Fließbedingungen eingehalten werden und keine oder nur eine geringe Wechselwirkung zwischen dem Flüssigbetonprodukt und dem Gas besteht. Es kann auch von Vorteil sein, wenn mehrere separate Bereiche der Oberfläche des Frischbetonproduktes mit Überdruck oder Unterdruck beaufschlagt werden. Auf diese Weise können inhomogen aufgebaute Produkte vermessen werden. So bestehen z.B. Pflastersteine im Allgemeinen aus einer Kernkomponente und einer Vorsatzkomponente, die aus unterschiedlichem Beton bestehen. Bei geeigneter Wahl der Druckbereiche kann die Gaspermeabilität für die beiden Komponenten separat bestimmt werden.

In einer Ausführungsform des Verfahrens wird die Druckdifferenz erzeugt, indem ein Bereich der Außenfläche des Frischbetonproduktes, für das das Maß für die Porosität bestimmt wird, mit einem Unterdruck beaufschlagt wird.

In einer Ausführungsform des Verfahrens wird der Unterdruck entweder mit Hilfe einer Vakuumpumpe mit vorbestimmter Pumpleistung erzeugt und aufrechterhalten, und als Maß für den Gasdurchfluss wird der durch die Vakuumpumpe erreichte Unterdruck gemessen, oder der Unterdruck wird mit Hilfe einer Vakuumpumpe erzeugt, und als Maß für den Gasdurchfluss wird die Geschwindigkeit des Druckanstiegs nach Deaktivierung der Vakuumpumpe gemessen. Wird zur Erzeugung und Aufrechterhaltung des Unterdrucks eine Vakuumpumpe verwendet, die mit einer definierten Pumpleistung betrieben wird, kann die für den Gasdurchfluss charakteristische Größe durch den durch die Vakuumpumpe erreichten Unterdruck gebildet werden. Je höher bei definierter Pumpleistung der Gasdurchfluss ist, desto schlechter ist das durch die Vakuumpumpe erreichte Vakuum. Alternativ kann die für den Gasdurchfluss charakteristische Größe auch durch die Zeitkonstante gebildet werden, mit der der Druck nach Erzeugung eines definierten Unterdrucks und Deaktivierung der Vakuumpumpe ansteigt. Dabei erfolgt der Druckanstieg umso schneller, je höher der Gasdurchfluss ist.

In einer Ausführungsform des Verfahrens wird die Messeinrichtung auf eine Außenfläche des Frischbetonproduktes, für das das Maß für die Porosität bestimmt wird, positioniert. Die Messeinrichtung kann sich beispielsweise bei Nichtgebrauch in einer Warteposition außerhalb des Bereichs, in dem es zu einer starken Bildung von Sprühnebel durch das Aufbringen des Beschichtungsmaterials kommt, befinden. Nach Abschluss des Sprühvorgangs und bevor ein neues Frischbetonprodukt bzw. eine neue Anzahl von Frischbetonprodukten beschichtet werden soll, wird die Messeinrichtung zur Messung in die eigentliche Messposition gebracht. Bevorzugt ist die Außenfläche auf die die Messeinrichtung positioniert wird, eine weitestgehend waagerechte Fläche. Eine waagerechte Fläche vereinfacht den Positioniervorgang der Messeinrichtung auf der Fläche. Es ist auch denkbar, dass die Messeinrichtung auf verschiedene Bereiche des Frischbetonproduktes, für das das Maß für die Porosität bestimmt wird, positioniert wird, um Messungen an unterschiedlichen Stellen durchzuführen. Das Maß für die Porosität kann dann bevorzugt ein Mittelwert der mehreren Messungen sein.

In einer Ausführungsform des Verfahrens wird aus dem Maß für die Porosität des Frischbetonproduktes eine auf die oder eine Anzahl von Frischbetonprodukten aufzutragende Auftragsmenge des Beschichtungsmaterials bestimmt.

In einer Ausführungsform des Verfahrens wird ein Maß für die Porosität aus einer vorbestimmten Beziehung zwischen der gemessenen Gaspermeabilität und einer Zuordnungstabelle oder zwischen der Gaspermeabilität und einer Zuordnungsfunktion bestimmt. Eine Zuordnungstabelle kann beispielsweise durch die Durchführung einer Testreihe, bei der die Gaspermeabilität mehrerer Frischbetonprodukte mit unterschiedlicher Porosität gemessen wird und nach Ende der Trockenzeit die finale Porositäten der Fertigbetonprodukte durch eine komplementäre Technik bestimmt wird, gewonnen werden. Die so erhaltenen Datenpaare stellen eine Zuordnungstabelle dar. Basierend auf den Daten der Zuordnungstabelle kann in einem nächsten Schritt eine Regression mit einer geeigneten Funktion, beispielsweise eine polynomische Funktion, durchgeführt werden. Mit der so gewonnenen Zuordnungsfunktion ist es möglich auch vorher nicht gemessene Werte der Gaspermeabilität einem Maß für die Porosität zuzuordnen. Eine alternative Möglichkeit ist beispielsweise die im Stand der Technik bekannten Verfahren zur Bestimmung der Porosität von Beton zu verwenden und davon ausgehend einen Zusammenhang mit der Gaspermeabilität herzustellen.

In einer Ausführungsform des Verfahrens wird mindestens ein Verfahrensparameter des Sprühvorgangs so gesteuert, dass die Auftragsmenge mit zunehmender Porosität des Frischbetonproduktes, für das das Maß für die Porosität bestimmt wird, zunimmt. Mit zunehmender Porosität steigt die zum Verschließen der Poren der Außenfläche benötigte Menge des Beschichtungsmaterials. Die beim Sprühen auf das Frischbetonprodukt aufgebrachte Menge kann beispielsweise durch eine Erhöhung der Durchflussmenge während des Sprühvorgangs oder eine Verringerung der Geschwindigkeit, mit der die Sprühvorrichtung über das Frischbetonprodukt bewegt wird, erreicht werden.

In einer Ausführungsform des Verfahrens werden während des Sprühvorgangs die Anzahl von Frischbetonprodukten und die Sprühvorrichtung relativ zueinander bewegt. Dabei können sich nur die Anzahl von Frischbetonprodukten, nur die Sprühvorrichtung oder sowohl die Anzahl von Frischbetonprodukten als auch die Sprühvorrichtung relativ zueinander bewegen. Eine relative Bewegung der Anzahl von Frischbetonprodukten zur Sprühvorrichtung kann auf unterschiedliche Weisen realisiert werden: beispielsweise kann nur die Anzahl von Frischbetonprodukten oder die Sprühvorrichtung, oder beide gleichzeitig bewegt werden. Eine Bewegung nur der Anzahl von Frischbetonprodukten ist beispielweise denkbar, wenn sie durch einen Sprühtunnel oder unterhalb eines Sprühbalkens mittels einer Förderanlage bewegt wird. Eine Bewegung nur der Sprühvorrichtung ist denkbar, wenn sich die Sprühvorrichtung an einem beweglichen Arm, wie beispielsweise einem Roboterarm, befindet und sie damit um die sich in Ruhe befindende Anzahl von Frischbetonprodukten herumbewegt wird. Eine weitere Möglichkeit, bei der sich sowohl die Sprühvorrichtung als auch die Anzahl von Frischbetonprodukten bewegen, kann eine Kombination der ersten beiden Möglichkeiten in Form einer Förderanlage sein, auf der sich die Anzahl von Frischbetonprodukten bewegt, und einer Sprühvorrichtung an einem Roboterarm, die um die sich bewegende Anzahl von Frischbetonprodukten herumbewegt wird.

In einer Ausführungsform des Verfahrens wird die Auftragsmenge gesteuert, indem die relative Geschwindigkeit der Bewegung der Sprühvorrichtung zu der Anzahl von Frischbetonprodukten variiert wird. Vorzugsweise wird die Auftragsmenge gesteuert, indem die Durchflussgeschwindigkeit des Beschichtungsmaterials konstant gehalten wird und die relative Geschwindigkeit der Bewegung der Sprühvorrichtung zur Anzahl von Frischbetonprodukten variiert wird.

In einer Ausführungsform des Verfahrens wird die Auftragsmenge gesteuert, indem die Durchflussgeschwindigkeit des Beschichtungsmaterials variiert wird. Die Durchflussgeschwindigkeit kann dabei beispielsweise die Geschwindigkeit sein, mit der das Beschichtungsmaterial durch die Sprühvorrichtung fließt, bevor es die Sprühvorrichtung verlässt. Vorzugsweise wird die Auftragsmenge gesteuert, indem die Durchflussgeschwindigkeit des Beschichtungsmaterials variiert wird und die relative Geschwindigkeit der Bewegung der Sprühvorrichtung zu der Anzahl von Frischbetonprodukten konstant gehalten wird. Weiter vorzugsweise ist auch eine Kombination der Variation der Durchflussgeschwindigkeit des Beschichtungsmaterials und der Variation der relativen Geschwindigkeit der Bewegung der Sprühvorrichtung zu der Anzahl von Frischbetonprodukten zur Auftragung der gewünschten Auftragsmenge des Beschichtungsmaterials denkbar.

Zur Durchführung des erfindungsgemäßen Verfahrens kommt eine Vorrichtung zum Einsatz, die folgende Elemente aufweist: eine Messeinrichtung zur Bestimmung eines Maßes für die Porosität eines Frischbetonproduktes, die angepasst ist, um ein Messsignal zu liefern, das für die Porosität des Frischbetonprodukts kennzeichnend ist, eine Sprühvorrichtung zum Aufbringen eines Beschichtungsmaterials, wie bevorzugt eines Oberflächenschutzproduktes, auf eine Oberfläche einer Anzahl von Frischbetonprodukten, und eine Steuervorrichtung, die mit der Messeinrichtung und der Sprühvorrichtung verbunden und angepasst ist, um das Messsignal zu empfangen und in Abhängigkeit von dem Messsignal die Auftragsmenge des Beschichtungsmaterials auf die Anzahl von Frischbetonprodukten zu steuern.

Diese Ausgestaltung hat den Vorteil, dass durch eine individuelle Messung der Porosität z.B. eines einzelnen Frischbetonproduktes oder einer Anzahl von Frischbetonprodukten die Variationen der Porosität bei der Bestimmung der eingesetzten Menge des Beschichtungsmaterials berücksichtigt werden kann. Diese Berücksichtigung der individuellen Porosität erlaubt eine kosteneffiziente Anpassung der eingesetzten Menge des Beschichtungsmaterials. Zudem ist der hohe Grad der Automation weit weniger arbeitsintensiv als eine manuelle Bestimmung der Auftragsmenge, was ebenfalls Personalkosten senken kann. Desweiteren ist die automatisierte, objektive Messung und Bestimmung der Auftragsmenge einfacher, schneller und zuverlässiger als eine manuelle, von den Fähigkeiten und Erfahrungen des jeweiligen Maschinenführers abhängige Bestimmung der Auftragsmenge.

In einer Ausführungsform der Vorrichtung wählt die Steuervorrichtung auf Basis des Messsignals der Messeinrichtung die Auftragsmenge des Beschichtungsmaterials aus und steuert die Verfahrensparameter des Sprühvorgangs zur Auftragung der zuvor bestimmten Auftragsmenge.

In einer Ausführungsform weist die Vorrichtung ferner einen beweglichen Arm mit zwei Haltevorrichtungen auf, wobei die erste Haltevorrichtung die Messeinrichtung und die zweite Haltevorrichtung die Sprühvorrichtung hält, und wobei die Steuervorrichtung mit dem Arm und den Haltevorrichtungen verbunden ist und die Bewegungen des Arms und der Messeinrichtung, insbesondere die Positionierung der Messeinrichtung auf einer Au-βenfläche des Frischbetonprodukts und der Sprühvorrichtung steuert. Der bewegliche Arm kann beispielsweise als ein Roboterarm ausgebildet sein. An diesem Roboterarm können die Haltevorrichtungen mit der Sprühvorrichtung und der Messeinrichtung an einem drehbaren Kopf des beweglichen Arms befestigt sein. Je nach Arbeitsschritt kann der bewegliche Kopf so gedreht werden, dass entweder die Messeinrichtung oder Sprühvorrichtung verwendet werden kann.

Diese Ausgestaltung hat den Vorteil, dass das Beschichtungsmaterial im Vergleich zu beispielsweise einem Sprühbalken oder einem Sprühtunnel auch auf komplexere Formen des Frischbetonprodukts bzw. der Anzahl von Frischbetonprodukten aufgrund der hohen Beweglichkeit des beweglichen Arms aufgebracht werden kann. So können beispielsweise weiter von der Sprühvorrichtung entfernt liegende Abschnitte eines einzelnen Frischbetonproduktes oder einer Anzahl von Frischbetonprodukten von näher an der Sprühvorrichtung liegenden und größeren Abschnitten bei der Aufbringung des Beschichtungsmaterials mit einem Sprühbalken oder in einem Sprühtunnel abgeschattet werden. Diese abgeschatteten Abschnitte können aber aufgrund der hohen Beweglichkeit eines beweglichen Roboterarms mit dem Beschichtungsmaterial besprüht werden.

Desweiteren sind bei einer Anbringung der zwei Haltevorrichtungen an einem drehbaren Kopf sowohl die Messeinrichtung als auch die Sprühvorrichtung nahe am zu vermessenden bzw. zu besprühenden Frischbetonprodukt. Im Vergleich zu einer Ausführungsform, in der zwei bewegliche Arme eingesetzt werden, wobei ein Arm die Messeinrichtung und ein zweiter Arm die Sprühvorrichtung aufweist, werden Totzeiten vermieden, in denen das Frischbetonprodukt nicht bearbeitet werden kann. Diese Totzeiten können beispielsweise durch das jeweilige Verfahren der beweglichen Arme entstehen. Auch stellt der Einsatz nur eines beweglichen Arms eine erhebliche Kostenersparnis bei der Anschaffung eines solchen Arms dar.

In einer Ausführungsform weist die Vorrichtung ferner einen beweglichen Arm mit einer Haltevorrichtung auf, wobei die Haltevorrichtung die Messeinrichtung hält, und bei der die Sprühvorrichtung als Sprühbalken oder Sprühtunnel mit mindestens einer Sprühdüse ausgebildet ist, und wobei die Steuervorrichtung mit dem Sprühbalken und dem Arm und der Haltevorrichtung verbunden ist und die Steuervorrichtung die Bewegungen des Arms und der Messeinrichtung, insbesondere zur Positionierung der Messeinrichtung auf einer Außenfläche des Frischbetonprodukts, steuert.

In einer bevorzugten Ausführungsform weist der Sprühbalken zwei oder mehr Sprühdüsen auf. Der bewegliche Arm kann beispielsweise als Roboterarm ausgestaltet sein, und sich am Sprühbalken, daneben oder davon losgelöst befinden. Beispielsweise kann der Sprühbalken oder der Sprühtunnel ein Teil einer Förderanlage sein, die die Anzahl von Frischbetonprodukten unter dem Sprühbalken oder durch den Sprühtunnel bewegt. Es ist auch denkbar, dass sich die Messeinrichtung losgelöst vor dem Sprühbalken bzw. Sprühtunnel befindet, so dass zuerst die Porosität der Außenfläche eines einzelnen Frischbetonprodukts oder einer Anzahl von Frischbetonprodukten im Stillstand oder in Bewegung bestimmt werden kann und anschließend das Beschichtungsmaterial während des Unterquerens des Sprühbalkens oder des Durchqueren des Sprühtunnels auf die Anzahl von Frischbetonprodukten aufgebracht werden kann. Es ist auch denkbar, dass sich der bewegliche Arm mit der Messeinrichtung mit der sich auf der Förderanlage bewegenden Anzahl von Frischbetonprodukten mit bewegen kann, so dass es während der Messung keine oder nur eine sehr geringe Relativbewegung zwischen der Messeinrichtung und der Anzahl von Frischbetonprodukten gibt. Nachdem die Messung beendet ist, fährt die Haltevorrichtung mit der Messeinrichtung zur nächsten Anzahl von Frischbetonprodukten zurück. Dieser Aufbau hat den Vorteil, dass die Förderanlage kontinuierlich betrieben werden kann.

In einer Ausführungsform der Vorrichtung ist die Messeinrichtung zur Bestimmung der Porosität angepasst, um die Gaspermeabilität gegenüber Luft zu bestimmen. Die Gaspermeabilität ist ein Maß für die Gefügewerte oder auch die Nutzporosität des Frischbetonproduktes. Die Gaspermeabilität ist als Permeabilität für ein bestimmtes Gas oder Gasgemisch zu verstehen, da sich die Permeabilität im Allgemeinen für verschiedene Gase bzw. Gasgemische unterscheidet. Für die Bestimmung des Vorhersagemodells und die späteren Messungen muss daher im Allgemeinen stets dasselbe Gas bzw. Gasgemisch verwendet werden. Das genaue Messverfahren ist dabei nicht von Bedeutung. Es kommt lediglich darauf an, dass stets dasselbe Messverfahren in demselben Verfahrensschritt, d.h. an vergleichbaren Frischbetonprodukten und Positionen auf den Frischbetonprodukten, durchgeführt wird.

Dabei ist es bevorzugt, wenn die Gaspermeabilität die Permeabilität gegenüber Luft ist. Diese Messung lässt sich auf besonders einfache, schnelle und kostengünstige Weise realisieren, da mit der normalen Atmosphäre gearbeitet werden kann und der Einsatz besonderer Gase nicht erforderlich ist. Es ist aber zu bedenken, dass in diesem Fall für die Genauigkeit der Messung eine obere Grenze existiert, da Beton gegenüber dem in der Luft enthaltenen Kohlendioxid nicht inert ist. Reicht die Genauigkeit der Messung mit Luft nicht aus, so können Gase wie Sauerstoff oder Stickstoff verwendet werden.

In einer Ausführungsform der Vorrichtung weist die Messeinrichtung zur Bestimmung der Porosität eine Kammer auf, die eine Öffnung hat, deren Ränder dichtend in Anlage an einen Bereich einer Außenfläche eines Frischbetonproduktes gebracht werden kann, um die Öffnung zu schließen, und bei der die Messeinrichtung ferner eine Druckerzeugungseinrichtung, mit der in der Kammer ein Überdruck oder ein Unterdruck erzeugt werden kann, und eine Einrichtung zur Bestimmung eines Maßes für den durch die Druckdifferenz bewirkten Gasdurchfluss durch das Frischbetonprodukt enthält. Mit anderen Worten kann mit der Druckerzeugungseinrichtung ein Überdruck oder ein Unterdruck in einem Raum hergestellt werden, der in der Messposition einseitig durch eine Außenfläche eines Frischbetonprodukts geschlossen wird. Die Einrichtung zur Bestimmung der Gaspermeabilität kann auch in vorteilhafter Weise mehrere separate dieser Kammern aufweisen, so dass es möglich ist, ein inhomogenes Frischbetonprodukt zu vermessen. So weisen beispielsweise Pflastersteine im Allgemeinen eine Kernkomponente und eine Vorsatzkomponente auf, die aus unterschiedlichem Beton bestehen. Mit zwei Messkammern kann dann bei geeigneter Anordnung die Gaspermeabilität separat für die beiden Komponenten bestimmt werden.

In einer Ausführungsform der Vorrichtung ist die Druckerzeugungseinrichtung angepasst, um in der durch das Frischbetonprodukt abgeschlossenen Kammer einen Unterdruck zu erzeugen.

In einer Ausführungsform der Vorrichtung umfasst die Druckerzeugungseinrichtung eine Vakuumpumpe mit einer vorbestimmten Pumpleistung und die Messeinrichtung umfasst zur Bestimmung der Gaspermeabilität eine Druckmeßeinrichtung zur Bestimmung des Druckes in der Kammer bei einer kontinuierlichen Druckerzeugung, oder die Druckerzeugungseinrichtung umfasst eine Vakuumpumpe und die Messeinrichtung enthält zur Bestimmung der Gaspermeabilität eine Zeitmesseinrichtung und eine Druckmeßeinrichtung, mit der die Geschwindigkeit des Druckanstiegs nach Deaktivierung der Vakuumpumpe gemessen werden kann. Mit anderen Worten umfasst die Druckerzeugungseinrichtung eine Vakuumpumpe, und die Einrichtung zur Bestimmung der Gaspermeabilität weist eine Druckmesseinrichtung zur Bestimmung des Druckes in der Kammer auf. Wird die Vakuumpumpe mit einer definierten Pumpleistung betrieben, so hängt der von der Druckmesseinrichtung gemessene Druck von dem durch den Unterdruck bewirkten Gasdurchfluss durch das Frischbetonprodukt ab. Ferner kann eine Zeitmesseinrichtung vorgesehen sein, mit der die Geschwindigkeit des Druckanstiegs nach Deaktivierung der Vakuumpumpe gemessen werden kann. Diese Zeitkonstante von einem definierten Druckniveau aus ist ein einfach und genau zu bestimmendes Maß für den Gasdurchfluss, da es nicht auf die genaue absolute Messung eines Drucks ankommt und daher geringere Anforderungen an die Druckmesseinrichtung gestellt werden.

In einer Ausführungsform der Vorrichtung steuert bzw. variiert die Steuervorrichtung die Geschwindigkeit der Bewegung der Sprühvorrichtung relativ zu der Anzahl von Frischbetonprodukten. Vorzugsweise wird die Auftragsmenge gesteuert, indem die Durchflussgeschwindigkeit des Beschichtungsmaterials konstant gehalten wird und die relative Geschwindigkeit der Bewegung der Sprühvorrichtung zur Anzahl von Frischbetonprodukten variiert wird.

In einer Ausführungsform der Vorrichtung variiert die Steuervorrichtung die Durchflussgeschwindigkeit des Beschichtungsmaterials. Dies ist eine Möglichkeit, um die gewünschte Auftragsmenge auf die Anzahl von Frischbetonprodukten aufzubringen. Die Durchflussgeschwindigkeit kann dabei beispielsweise die Geschwindigkeit sein, mit der das Beschichtungsmaterial durch die Sprühvorrichtung fließt, bevor es die Sprühvorrichtung verlässt. Vorzugsweise steuert die Steuervorrichtung die Auftragsmenge, indem die Steuervorrichtung die Durchflussgeschwindigkeit des Beschichtungsmaterials variiert und die relative Geschwindigkeit der Bewegung der Sprühvorrichtung zur Anzahl von Frischbetonprodukten konstant hält. Weiter vorzugsweise ist auch eine Kombination der Variation der Durchflussgeschwindigkeit des Beschichtungsmaterials und der Variation der relativen Geschwindigkeit der Bewegung der Sprühvorrichtung zur Anzahl von Frischbetonprodukten zur Auftragung der gewünschten Auftragsmenge des Beschichtungsmaterials denkbar. Im Folgenden wird die Erfindung anhand von lediglich Ausführungsformen zeigenden Zeichnungen weiter erläutert. Diese Abbildungen sind beispielhaft zu verstehen und schränken die Erfindung nicht ein. Dabei zeigt
- Figur 1: eine Schnittansicht einer Ausführungsform der Messeinrichtung,
- Figur 2: eine perspektivische Darstellung einer Ausführungsform der Vorrichtung mit einem Roboterarm, an dessen beweglichem Ende sich die Messeinrichtung und die Sprühvorrichtung befinden,
- Figur 3: eine seitliche Ansicht eines drehbaren Kopfes eines Roboterarms mit der Messeinrichtung und der Sprühvorrichtung,
- Figur 4: eine weitere seitliche Ansicht eines drehbaren Kopfes eines Roboterarms mit der Messeinrichtung und der Sprühvorrichtung,
- Figur 5: eine perspektivische Darstellung einer Ausführungsform der Vorrichtung, bei der ein Frischbetonprodukt auf einer Förderanlage unter einem Sprühbalken gefahren wird.

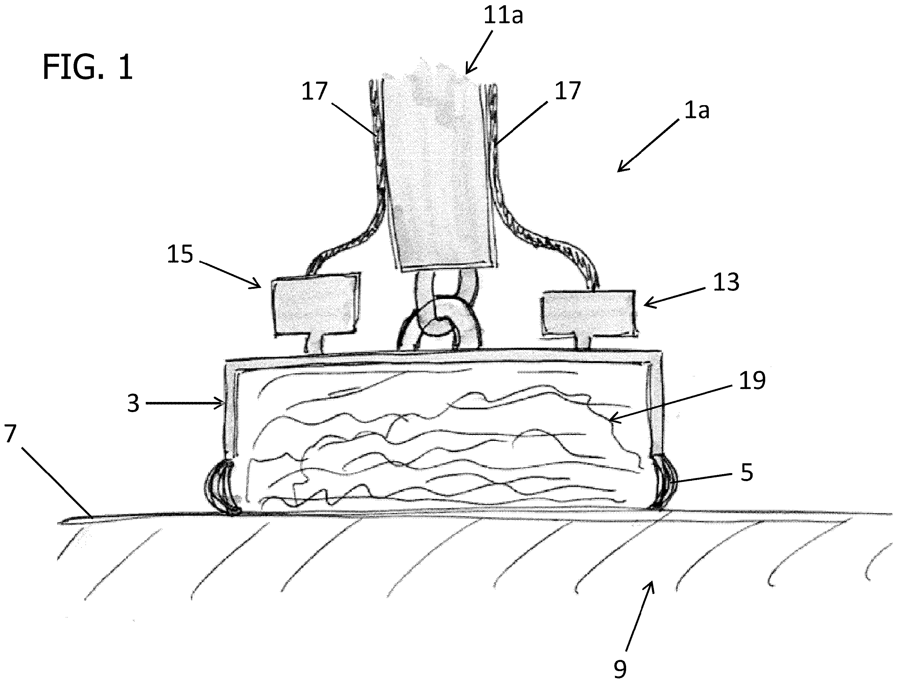

Figur 1 zeigt eine Schnittansicht einer Ausführungsform der Messeinrichtung 1a mit einer Kammer 3, die durch eine Gummidichtung dichtend mit der Außenfläche 7 eines Frischbetonprodukts 9 in Anlage gebracht ist. Die Kammer 3 ist mit einer beweglichen Haltevorrichtung 11a verbunden. Zusätzlich weist die Kammer eine Druckerzeugungseinrichtung 13 sowie eine Druckmeßeinrichtung 15 auf. Sowohl die Druckerzeugungseinrichtung 13 als auch die Druckmeßeinrichtung 15 sind über Verbindungsleitungen 17 mit der Steuervorrichtung (nicht gezeigt) verbunden.

Wird nun eine Messung eines Maßes der Porosität, d.h. eine Messung der Gaspermeabilität, durchgeführt, beaufschlagt die Druckerzeugungseinrichtung 13 die in dichtender Anlage mit der Außenfläche 7 des Frischbetonproduktes 9 gebrachte Kammer 3 mit einem Unterdruck. Die Folge ist ein Druckanstieg in der Kammer 3 aufgrund von durch die Poren des Betons strömende Luft 19. Der Druckanstieg wird dabei von der Druckmeßeinrichtung 15 gemessen.

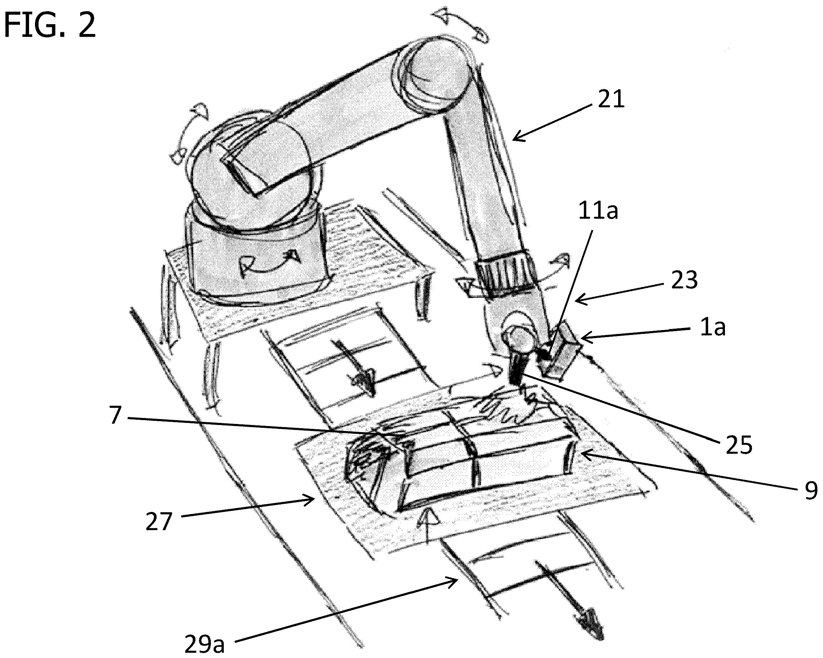

Figur 2 zeigt eine perspektivische Darstellung einer Ausführungsform einer Vorrichtung zum Aufbringen eines Beschichtungsmaterials in Form eine Oberflächenschutzproduktes auf Frischbetonprodukte - bzw. letztendlich Betonwarenprodukte. Die Vorrichtung weist einen Roboterarm 21 auf. An dem beweglichen Ende des Roboterarms 21 befindet sich ein drehbarer Kopf 23. An dem drehbaren Kopf 23 befindet sich wiederum jeweils über Haltevorrichtungen 11a die Messeinrichtung 1a und die Sprühvorrichtung 25. Der drehbare Kopf 23 ist so ausgestaltet, dass entweder die Messeinrichtung 1a oder die Sprühvorrichtung 25 verwendet werden kann. Wird eine Vorrichtung nicht verwendet, befindet sie sich in einem festen Winkel zur jeweils anderen Vorrichtung. Die Messeinrichtung 1a, die Sprühvorrichtung 25 sowie der Roboterarm 21 sind jeweils mit der Steuervorrichtung verbunden (nicht gezeigt).

Zur Ausführung des erfindungsgemäßen Verfahrens wird nun ein Frischbetonprodukt 9 mittels eines Förderwagens 27 einer Förderanlage 29a in die gewünschte Position vor den Roboterarm 21 gefahren. In einem nächsten Schritt steuert die Steuervorrichtung (nicht gezeigt) die Bewegung des Roboterarms 21 und seines drehbaren Kopfes 23 und positioniert die Messeinrichtung 1a auf einer Außenfläche 7 des Frischbetonproduktes 9. Die Messeinrichtung 1a wird mit der Außenfläche 7 in dichtender Anlage gebracht und eine Druckerzeugungseinrichtung 13 beaufschlägt die Kammer 3 mit einem Unterdruck. In Folge des Unterdrucks strömt Luft durch die Poren des Frischbetonprodukts 9 in die Kammer 3. Der damit verbundene Druckanstieg wird mit der Druckmeßeinrichtung 15 gemessen. Die dabei gewonnenen Messwerte werden von der Steuervorrichtung (nicht gezeigt) ausgewertet und die erforderliche Auftragsmenge auf das Frischbetonprodukt 9 wird bestimmt. Im nächsten Schritt steuert die Steuervorrichtung (nicht gezeigt) die Bewegung des Roboterarms 21 sowie des drehbaren Kopfes 23, hebt dabei die Messeinrichtung 1a von der Außenfläche 7 des Frischbetonproduktes 9 ab und dreht die Sprühvorrichtung 25 in eine Ausführposition. Beim darauf folgenden Sprühvorgang wird die Sprühvorrichtung über den Roboterarm 21 in einer vorbestimmten Weise über die Außenflächen 7 des Frischbetonproduktes 9 bewegt. Nachdem alle Außenflächen 7 mit dem Oberflächenschutz bedeckt sind, wird das Frischbetonprodukt 9 mit Hilfe der Förderanlage 29a weitergefahren, die Sprühvorrichtung wird selbstständig gesäubert und ein neues Frischbetonprodukt 9 wird vor den Roboterarm 21 gefahren.

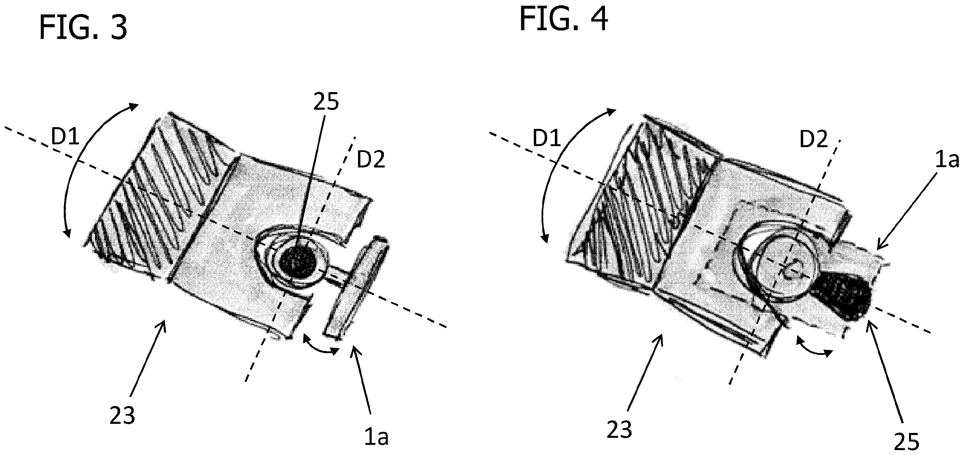

In Figur 3 ist eine seitliche Ansicht des drehbaren Kopfes 23 in einer Stellung, bei der die Kammer 3 der Messeinrichtung 1a senkrecht zur Drehachse D1 ausgerichtet ist, gezeigt. Die sich ebenfalls an dem drehbaren Kopf 23 befindende Sprühvorrichtung 25 ist senkrecht zur Messeinrichtung 1a am drehbaren Kopf angebracht und zeigt aus der Blattebene in Richtung des Betrachters. Figur 4 zeigt die gleiche seitliche Ansicht des drehbaren Kopfes 23, wobei in diesem Fall die Sprühvorrichtung 25 in Richtung der Drehachse D1 des drehbaren Kopfes 23 zeigt und im Vergleich zu Figur 3 um die Drehachse D2 gedreht ist. Durch die Anbringung der Messeinrichtung 1a und der Sprühvorrichtung 25 an demselben drehbaren Kopf 23 ist eine schnelle Abfolge der Arbeitsschritte Messen und Sprühen möglich. Desweiteren wird der Aufbau der Vorrichtung vereinfacht, da keine zwei Roboterarme zur Bewegung der Messeinrichtung 1a und der Sprühvorrichtung 25 benötigt werden.

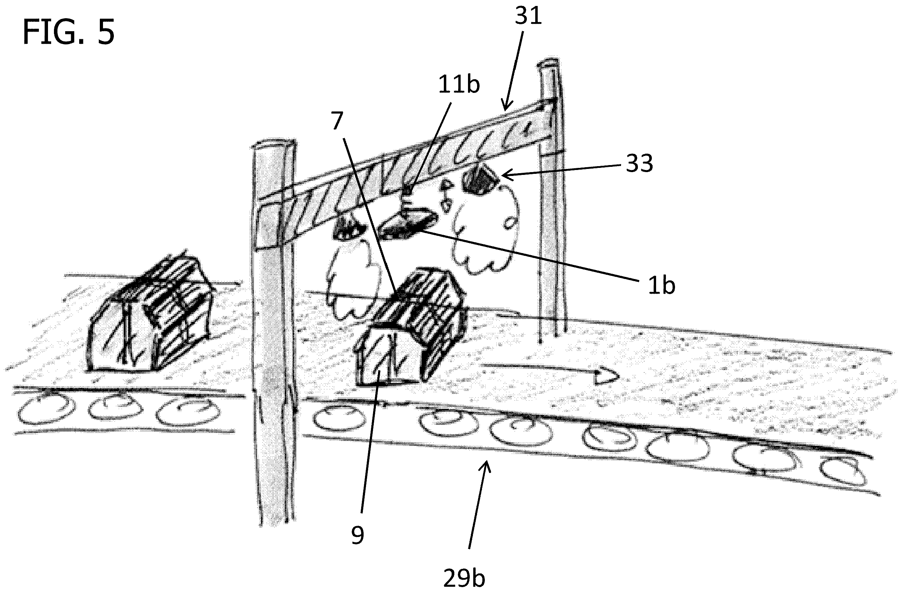

In Figur 5 ist eine weitere Ausführungsform der erfindungsgemäßen Vorrichtung zum Aufbringen eines Beschichtungsmaterials in Form eine Oberflächenschutzproduktes auf Frischbetonprodukte - bzw. letztendlich Betonwarenprodukte - in einer perspektivischen Darstellung gezeigt. In dieser Ausführungsform wird ein Frischbetonprodukt 9 auf einer Förderanlage 29b unter einen Sprühbalken 31 hindurch gefahren. Der Sprühbalken weist in diesem Fall zwei Sprühdüsen 33 auf, sowie eine Haltevorrichtung 11b mit der Messeinrichtung 1b.

Zur Ausführung des erfindungsgemäßen Verfahrens wird nun ein Frischbetonprodukt 9 mittels der Förderanlage 29b unter den Sprühbalken gebracht. In einem nächsten Schritt steuert die Steuerung (nicht gezeigt) die Bewegung und Positionierung der Messeinrichtung 1b auf einer Außenfläche 7 des Frischbetonproduktes 9. Die Messeinrichtung 1b wird mit der Außenfläche 7 in dichtender Anlage gebracht und eine Druckerzeugungseinrichtung 13 beaufschlägt die Kammer 3 mit einem Unterdruck. In Folge des Unterdrucks strömt Luft durch die Poren des Frischbetonprodukts 9 in die Kammer 3. Der damit verbundene Druckanstieg wird mit der Druckmeßeinrichtung 15 gemessen. Die dabei gewonnenen Messwerte werden von der Steuervorrichtung (nicht gezeigt) ausgewertet und die erforderliche Auftragsmenge auf das Frischbetonprodukt 9 wird bestimmt. Im nächsten Schritt steuert die Steuervorrichtung (nicht gezeigt) die Bewegung der Haltevorrichtung 11b und hebt dabei die Messeinrichtung 1b von der Außenfläche 7 des Frischbetonprodukts 9. Beim darauf folgenden Sprühvorgang steuert die Steuervorrichtung (nicht gezeigt) die Durchflussmenge durch die Sprühdüsen 33 des Sprühbalkens 31. Nachdem alle Außenflächen 7 mit dem Oberflächenschutz bedeckt sind, wird das Frischbetonprodukt 9 mit Hilfe der Förderanlage 29b weitergefahren, die Sprühvorrichtung säubert sich selbstständig und ein neues Frischbetonprodukt 9 wird zum Sprühbalken 31 gefahren.

Es ist auch denkbar, dass sich die Messeinrichtung 1b vor dem Sprühbalken befindet, so dass zuerst die Porosität der Außenfläche 7 des Frischbetonprodukts 9 im Stillstand bestimmt wird und anschließend das Oberflächenschutzprodukt in Bewegung, während des Unterquerens des Sprühbalkens 31, auf das Frischbetonprodukt 9 aufgebracht wird. Es ist auch denkbar, dass sich die Haltevorrichtung 11b der Messeinrichtung 1b mit dem sich auf der Förderanlage 29b bewegenden Frischbetonprodukt 9 mit bewegen kann, so dass es während der Messung keine oder nur eine sehr geringe Relativbewegung zwischen der Messeinrichtung 1b und dem Frischbetonprodukt 9 gibt. Nachdem die Messung beendet ist, fährt die Haltevorrichtung 11b mit der Messeinrichtung 1b zum nächsten Frischbetonprodukt 7 zurück. Dieser Aufbau hat den Vorteil, dass die Förderanlage kontinuierlich betrieben werden kann.

## Patentansprüche

1. Verfahren zum Versehen einer Anzahl von Betonwarenprodukten mit einem Beschichtungsmaterial, bei dem während eines Prozesses zur Herstellung von Betonwarenprodukten, der einen einer Frischbetonphase entsprechenden Zwischenschritt nach Formen und Verdichten aufweist, in dem die Betonwarenprodukte als Frischbetonprodukte (9) vorliegen, in der Frischbetonphase durch einen Sprühvorgang mittels einer Sprühvorrichtung (25) ein Beschichtungsmaterial auf eine Oberfläche einer Anzahl der Frischbetonprodukte (9) aufgebracht wird, die der Anzahl von Betonwarenprodukten entsprechen, wobei
- ein Maß für die Porosität eines Frischbetonproduktes (9) aus der Frischbetonphase über eine Messeinrichtung (1a, 1b) bestimmt wird und
- der Sprühvorgang des Beschichtungsmaterials auf die Anzahl von Frischbetonprodukten (9) in Abhängigkeit von dem bestimmten Maß für die Porosität durch Steuerung mindestens eines Verfahrensparameters zur Einstellung einer Auftragsmenge des Beschichtungsmaterials durchgeführt wird.

2. Verfahren nach Anspruch 1, bei dem das Maß für die Porosität eine Gaspermeabilität ist und bei dem die Gaspermeabilität gemessen wird, indem entlang einer Außenfläche (7) des Frischbetonproduktes (9), für das das Maß für die Porosität bestimmt wird, eine Druckdifferenz hergestellt und ein Maß für den durch die Druckdifferenz bewirkten Gasdurchfluss durch das Frischbetonprodukt (9) bestimmt wird.

3. Verfahren nach Anspruch 2, bei dem die Druckdifferenz erzeugt wird, indem ein Bereich der Außenfläche (7) des Frischbetonproduktes (9), für das das Maß für die Porosität bestimmt wird, mit einem Unterdruck beaufschlagt wird.

4. Verfahren nach Anspruch 3, bei dem
- der Unterdruck mit Hilfe einer Vakuumpumpe mit vorbestimmter Pumpleistung erzeugt und aufrechterhalten wird und als Maß für den Gasdurchfluss der durch die Vakuumpumpe erreichte Unterdruck gemessen wird, oder
- der Unterdruck mit Hilfe einer Vakuumpumpe erzeugt wird und als Maß für den Gasdurchfluss die Geschwindigkeit des Druckanstiegs nach Deaktivierung der Vakuumpumpe gemessen wird.

5. Verfahren nach einem der vorherigen Ansprüche, bei dem der mindestens eine Verfahrensparameter des Sprühvorgangs so gesteuert wird, dass die Auftragsmenge mit zunehmender Porosität des Frischbetonproduktes (9), für das das Maß für die Porosität bestimmt wird, zunimmt.

6. Verfahren nach einem der vorherigen Ansprüche, bei dem die Auftragsmenge gesteuert wird, indem die relative Geschwindigkeit der Bewegung der Sprühvorrichtung (25) zu der Anzahl von Frischbetonprodukten (9) variiert wird und/oder indem die Durchflussgeschwindigkeit des Beschichtungsmaterials variiert wird.

7. Vorrichtung zur Durchführung eines Verfahrens nach einem der vorherigen Ansprüche mit
- einer Messeinrichtung (1a, 1b) zur Bestimmung eines Maßes für die Porosität eines Frischbetonproduktes (9), die angepasst ist, um ein Messsignal zu liefern, das für die Porosität des Frischbetonprodukts (9) kennzeichnend ist,
- einer Sprühvorrichtung (25) zum Aufbringen eines Beschichtungsmaterials auf eine Oberfläche einer Anzahl von Frischbetonprodukten (9) und
- einer Steuervorrichtung, die mit der Messeinrichtung (1a, 1b) und der Sprühvorrichtung (25) verbunden und angepasst ist, um das Messsignal zu empfangen und in Abhängigkeit von dem Messsignal die Auftragsmenge des Beschichtungsmaterials auf die Anzahl von Frischbetonprodukten (9) zu steuern.

8. Vorrichtung nach Anspruch 7, bei dem die Steuervorrichtung auf Basis des Messsignals der Messeinrichtung (1a, 1b) die Auftragsmenge des Beschichtungsmaterials auswählt und die Verfahrensparameter des Sprühvorgangs zur Auftragung der zuvor bestimmten Auftragsmenge steuert.

9. Vorrichtung nach Anspruch 7 oder 8, die ferner einen beweglichen Arm (21) mit zwei Haltevorrichtungen (11a, 11b) aufweist, wobei die erste Haltevorrichtung die Messeinrichtung (1a, 1b) und die zweite Haltevorrichtung die Sprühvorrichtung (25) hält, und wobei die Steuervorrichtung mit dem Arm (21) und den Haltevorrichtungen verbunden ist und die Bewegungen des Arms (21) und der Messeinrichtung (1a, 1b), insbesondere die Positionierung der Messeinrichtung (1a, 1b) auf einer Außenfläche (7) des Frischbetonprodukts (9), und der Sprühvorrichtung (25) steuert.

10. Vorrichtung nach Anspruch 7 oder 8, die ferner einen beweglichen Arm (21) mit einer Haltevorrichtung (11a) aufweist, wobei die Haltevorrichtung die Messeinrichtung (1a, 1b) hält, und bei der die Sprühvorrichtung (25) als Sprühbalken (31) oder Sprühtunnel mit mindestens einer Sprühdüse (33) ausgebildet ist, und wobei die Steuervorrichtung mit dem Sprühbalken (31) und dem Arm (21) und der Haltevorrichtung verbunden ist und die Steuervorrichtung die Bewegungen des Arms (21) und der Messeinrichtung (1a, 1b), insbesondere zur Positionierung der Messeinrichtung (1a, 1b) auf einer Außenfläche (7) des Frischbetonprodukts (9), steuert.

11. Vorrichtung nach einem der Ansprüche 7 bis 10, bei der die Messeinrichtung (1a, 1b) zur Bestimmung der Porosität eine Kammer (3) aufweist, die eine Öffnung hat, deren Ränder dichtend in Anlage an einen Bereich einer Außenfläche (7) eines Frischbetonproduktes (9) gebracht werden kann, um die Öffnung zu schließen, und bei der die Messeinrichtung (1a, 1b) ferner eine Druckerzeugungseinrichtung (13), mit der in der Kammer (3) ein Überdruck oder ein Unterdruck erzeugt werden kann, und eine Einrichtung zur Bestimmung eines Maßes für den durch die Druckdifferenz bewirkten Gasdurchfluss durch das Frischbetonprodukt (9) enthält.

12. Vorrichtung nach Anspruch 11, bei der die Druckerzeugungseinrichtung (13) angepasst ist, um in der durch das Frischbetonprodukt (9) abgeschlossenen Kammer (3) einen Unterdruck zu erzeugen.

13. Vorrichtung nach Anspruch 12,
- bei der die Druckerzeugungseinrichtung (13) eine Vakuumpumpe mit einer vorbestimmten Pumpleistung umfasst und bei der die Messeinrichtung (1a, 1b) zur Bestimmung der Gaspermeabilität eine Druckmeßeinrichtung (15) zur Bestimmung des Druckes in der Kammer (3) bei einer kontinuierlichen Druckerzeugung aufweist, oder
- bei der die Druckerzeugungseinrichtung (13) eine Vakuumpumpe umfasst und die Messeinrichtung (1a, 1b) zur Bestimmung der Gaspermeabilität eine Zeitmesseinrichtung und eine Druckmesseinrichtung (15) enthält, mit der die Geschwindigkeit des Druckanstiegs nach Deaktivierung der Vakuumpumpe gemessen werden kann.

14. Vorrichtung nach einem der Ansprüche 7 bis 13, bei der die Steuervorrichtung die Geschwindigkeit der Bewegung der Sprühvorrichtung (25) relativ zu der Anzahl von Frischbetonprodukten (9) variiert.

15. Vorrichtung nach einem der Ansprüche 7 bis 13, bei der die Steuervorrichtung die Durchflussgeschwindigkeit des Beschichtungsmaterials variiert.

## Claims

1. Method of providing a number of concrete products with a coating material, in which during a process of making concrete products having an intermediate step after forming and compacting corresponding to a fresh concrete phase, in which the concrete products are present as fresh concrete products (9), a coating material is applied in the fresh concrete phase in a spraying process by means of a spray device (25) to a surface of a number of fresh concrete products (9) corresponding to the number of concrete products, wherein
- a measure for the porosity of a fresh concrete product (9) from the fresh concrete phase is determined via a measuring device (1a, 1b), and
- the spraying process of the coating material on the number of fresh concrete products (9) is conducted in dependence of the determined measure for the porosity by controlling at least one process parameter for adjusting an application rate of the coating material.

2. Method according to claim 1, wherein the measure for the porosity is a gas permeability and wherein the gas permeability is measured by establishing a pressure difference along an outer surface (7) of the fresh concrete product (9), for which the measure for the porosity is determined, and by determining a measure for the gas flow through the fresh concrete product (9) caused by the pressure difference.

3. Method according to claim 2, wherein the pressure difference is generated by applying a negative pressure to a portion of the outer surface (7) of the fresh concrete product (9) for which the measure for the porosity is determined.

4. Method according to claim 3,
- wherein the negative pressure is generated and maintained by means of a vacuum pump with a predetermined pumping capacity and wherein the negative pressure generated by the vacuum pump is measured as a measure for the gas flow, or
- wherein the negative pressure is generated by means of a vacuum pump and wherein a rate of the pressure rise after the deactivation of the vacuum pump is measured as a measure for the gas flow.

5. Method according to any of the preceding claims, wherein the at least one process parameter of the spraying process is controlled so that the application rate increases with increasing porosity of the fresh concrete product (9) for which the measure for the porosity is determined.

6. Method according to any of the preceding claims, wherein the application rate is controlled by varying the relative speed of the movement of the spray device (25) to the number of fresh concrete products (9) and/or by varying the flow rate of the coating material.

7. Apparatus for carrying out a method according to any of the preceding claims comprising
- a measuring device (1a, 1b) for determining a measure for the porosity of a fresh concrete product (9) adapted to provide a measuring signal which characterizes the porosity of the fresh concrete product (9),
- a spray device (25) for applying a coating material to a surface of a number of fresh concrete products (9), and
- a conctrol device which is connected to the measuring device (1a, 1b) and the spray device (25) and which is adapted to receive the measuring signal and to control in dependence of the measuring signal the application rate of the coating material on the number of fresh concrete products (9).

8. Apparatus according to claim 7, wherein the control device selects on the basis of the measuring signal of the measuring device (1a, 1b) the application rate of the coating material and controls the process parameters of the spraying process for application of the previously determined application rate.

9. Apparatus according to claim 7 or claim 8, further comprising a movable arm (21) with two holding devices (11a, 11b), wherein the first holding device holds the measuring device (1a, 1b) and the second holding device holds the spray device (25), and wherein the control device is connected with the arm (21) and the holding devices and controls the movements of the arm (21) and of the measuring device (1a, 1b), in particular the positioning of the measuring device (1a, 1b) on an outer surface (7) of the fresh concrete product (9), and of the spray device (25).

10. Apparatus according to claim 7 or claim 8, further comprising a movable arm (21) with a holding device (11a), wherein the holding device holds the measuring device (1a, 1b) and wherein the spray device (25) is provided as a spray bar (31) or a spray tunnel with at least one spray nozzle (33), and wherein the control device is connected with the spray bar (31) and the arm (21) and the holding device, and wherein the control device controls the movements of the arm (21) and of the measuring device (1a, 1b), in particular for positioning the measuring device (1a, 1b) on an outer surface (7) of the fresh concrete product (9).

11. Apparatus according to any of claims 7 to 10, wherein the measuring device (1a, 1b) for determining the porosity comprises a chamber (3) which has an opening whose edges can be sealingly brought into abutment with a region of an outer surface (7) of a fresh concrete product (9), to close the opening, and wherein the measuring device (1a, 1b) further comprises a pressure generating unit (13) with which a positive pressure or a negative pressure can be generated in the chamber (3), and a device for determining a measure for the gas flow through the fresh concrete product (9) caused by the pressure difference.

12. Apparatus according to claim 11, wherein the pressure generating means (13) is adapted to create a negative pressure in the chamber (3) closed by the fresh concrete product (9).

13. Apparatus according to claim 12,
- wherein the pressure generating means (13) comprises a vacuum pump with a predetermined pumping capacity and wherein the measuring device (1a, 1b) for determining the gas permeability comprises a pressure measuring unit (15) for determining the pressure in the chamber (3) during continuous pressure generation, or
- wherein the pressure generating means (13) comprises a vacuum pump, and the measuring device (1a, 1b) for determining the gas permeability comprises a time measuring device and a pressure measuring device (15) for measuring the rate of the pressure increase after deactivation of the vacuum pump.

14. Apparatus according to any of claims 7 to 13, wherein the control device varies the speed of the movement of the spray device (25) relative to the number of fresh concrete products (9).

15. Apparatus according to any of claims 7 to 13, wherein the control device varies the flow rate of the coating material.

## Revendications

1. Procédé permettant de pourvoir un nombre de produits en béton d'un matériau de revêtement, selon lequel, au cours d'un processus de fabrication de produits en béton qui comprend une étape intermédiaire, laquelle intervient après la mise en forme et le compactage et correspond à une phase de béton frais et lors de laquelle les produits en béton sont présents sous forme de produits en béton frais (9), on applique, dans la phase de béton frais, à l'aide d'une opération de pulvérisation au moyen d'un dispositif de pulvérisation (25), un matériau de revêtement sur une surface d'un certain nombre de produits en béton frais (9) qui correspondent au nombre de produits en béton, sachant que
- l'on détermine une mesure pour la porosité d'un produit en béton frais (9) provenant de la phase de béton frais, par l'intermédiaire d'un dispositif de mesure (1a, 1b), et
- l'opération de pulvérisation du matériau de revêtement sur le nombre de produits en béton frais (9) est exécutée en fonction de la mesure déterminée pour la porosité, en contrôlant au moins un paramètre de procédé pour régler une quantité appliquée du matériau de revêtement.

2. Procédé selon la revendication 1, selon lequel la mesure pour la porosité est une perméabilité aux gaz, et selon lequel la perméabilité aux gaz est mesurée en créant une différence de pression le long d'une surface extérieure (7) du produit en béton frais (9) pour lequel on détermine la mesure pour la porosité, et on détermine une mesure pour le débit de gaz provoqué par la différence de pression, à travers le produit en béton frais (9).

3. Procédé selon la revendication 2, selon lequel la différence de pression est produite en appliquant une dépression à une partie de la surface extérieure (7) du produit en béton frais (9) pour lequel on détermine la mesure pour la porosité.

4. Procédé selon la revendication 3, selon lequel
- la dépression est produite et maintenue à l'aide d'une pompe à vide à puissance de pompage prédéterminée, et on mesure, en tant que mesure pour le débit de gaz, la dépression atteinte par a pompe à vide, ou
- la dépression est produite à l'aide d'une pompe à vide, et on mesure, en tant que mesure pour le débit de gaz, la vitesse de l'augmentation de pression, après la désactivation de la pompe à vide.

5. Procédé selon une des revendications précédentes, selon lequel le paramètre de procédé, au nombre d'au moins un, de l'opération de pulvérisation est contrôlé de manière à ce que la quantité appliquée augmente à mesure que la porosité du produit en béton frais (9), pour lequel on détermine la mesure pour la porosité, augmente.

6. Procédé selon une des revendications précédentes, selon lequel la quantité appliquée est contrôlée en faisant varier la vitesse relative du mouvement du dispositif de pulvérisation (25) par rapport au nombre de produits en béton frais (9) et/ou en faisant varier le débit du matériau de revêtement.

7. Dispositif pour la mise en oeuvre d'un procédé selon une des revendications précédentes, comprenant
- un dispositif de mesure (1a, 1b) qui est destiné à déterminer une mesure pour la porosité d'un produit en béton frais (9) et qui est adapté pour fournir un signal de mesure caractéristique de la porosité du produit en béton frais (9),
- un dispositif de pulvérisation (25) destiné à appliquer un matériau de revêtement sur une surface d'un certain nombre de produits en béton frais (9), et
- un dispositif de commande qui est relié au dispositif de mesure (1a, 1b) et au dispositif de pulvérisation (25) et qui est adapté pour recevoir le signal de mesure et pour contrôler la quantité appliquée du matériau de revêtement sur le nombre de produits en béton frais (9), en fonction du signal de mesure.

8. Dispositif selon la revendication 7, dans lequel le dispositif de commande sélectionne la quantité appliquée du matériau de revêtement, sur la base du signal de mesure du dispositif de mesure (1a, 1b), et contrôle les paramètres de procédé de l'opération de pulvérisation, en vue de l'application de la quantité préalablement déterminée.

9. Dispositif selon la revendication 7 ou 8, qui présente en outre un bras (21) mobile doté de deux dispositifs de support (11a, 11b), le premier dispositif de support portant le dispositif de mesure (1a, 1b), et le deuxième dispositif de support portant le dispositif de pulvérisation (25), et le dispositif de commande étant relié au bras (21) et aux dispositifs de support et contrôlant les mouvements du bras (21) et du dispositif de mesure (1a, 1b), en particulier le positionnement du dispositif de mesure (1a, 1b) sur une surface extérieure (7) du produit en béton frais (9), et du dispositif de pulvérisation (25).

10. Dispositif selon la revendication 7 ou 8, qui présente en outre un bras (21) mobile doté d'un dispositif de support (11a), le dispositif de support portant le dispositif de mesure (1a, 1b), et dans lequel le dispositif de pulvérisation (25) est réalisé sous forme de rampe de pulvérisation (31) ou de tunnel de pulvérisation comportant au moins une buse de pulvérisation (33), et le dispositif de commande étant relié à la rampe de pulvérisation (31) et au bras (21) et au dispositif de support, et le dispositif de commande contrôlant les mouvements du bras (21) et du dispositif de mesure (1a, 1b), en particulier en vue du positionnement du dispositif de mesure (1a, 1b) sur une surface extérieure (7) du produit en béton frais (9).

11. Dispositif selon une des revendications 7 à 10, dans lequel, pour déterminer la porosité, le dispositif de mesure (1a, 1b) présente une chambre (3) qui comporte une ouverture dont les bords peuvent être amenés de manière étanche en appui contre une partie d'une surface extérieure (7) d'un produit en béton frais (9) pour fermer l'ouverture, et dans lequel le dispositif de mesure (1a, 1b) comprend en outre un dispositif de génération de pression (13), qui permet de créer une surpression ou une dépression dans la chambre (3), et un dispositif pour déterminer une mesure pour le débit de gaz provoqué par la différence de pression et traversant le produit en béton frais (9).

12. Dispositif selon la revendication 11, dans lequel le dispositif de génération de pression (13) est adapté pour produire une dépression dans la chambre (3) fermée par le produit en béton frais (9).

13. Dispositif selon la revendication 12,
- dans lequel le dispositif de génération de pression (13) comprend une pompe à vide avec une puissance de pompage prédéterminée, et dans lequel le dispositif de mesure (1a, 1b), pour déterminer la perméabilité aux gaz, présente un dispositif de mesure de pression (15) destiné à déterminer la pression dans la chambre (3), en présence d'une génération de pression continue, ou
- dans lequel le dispositif de génération de pression (13) comprend une pompe à vide, et le dispositif de mesure (1a, 1b), pour déterminer la perméabilité aux gaz, comporte un dispositif de mesure de temps et un dispositif de mesure de pression (15) qui permet de mesurer la vitesse de l'augmentation de pression après la désactivation de la pompe à vide.

14. Dispositif selon une des revendications 7 à 13, dans lequel le dispositif de commande fait varier la vitesse du mouvement du dispositif de pulvérisation (25) par rapport au nombre de produits en béton frais (9).

15. Dispositif selon une des revendications 7 à 13, dans lequel le dispositif de commande fait varier le débit du matériau de revêtement.
